# EUROPEAN PATENT SPECIFICATION

(11) **EP 4 107 178 B1**
(45) Date of publication and mention of the grant of the patent: **10.01.2024**
(21) Application number: 21704841.2
(22) Date of filing: 18.02.2021
(51) Int. Cl.: C07K 14/71, C07K 1/22, G01N 33/68

(54) **AFFINITY LIGAND FOR THE SOLUBLE PART OF FIBROBLAST GROWTH FACTOR RECEPTOR 3 (SFGFR3)**
AFFINITÄTSLIGANDE FÜR DEN LÖSLICHEN TEIL DES FIBROBLASTENWACHSTUMSFAKTORREZEPTORS 3 (SFGFR3)
LIGAND D'AFFINITÉ POUR LA PARTIE SOLUBLE DU RÉCEPTEUR 3 DU FACTEUR DE CROISSANCE DE FIBROBLASTES (SFGFR3)

(30) Priority: 21.02.2020 EP 20158721
(43) Date of publication of application: 28.12.2022
(73) Proprietor: NAVIGO PROTEINS GMBH, 06120 Halle/Saale (DE)
(72) Inventor: LOTZE, Jonathan, 06120 Halle/Saale (DE); MEYSING, Maren, 06120 Halle/Saale (DE); KAHL, Mathias, 06120 Halle/Saale (DE); KNICK, Paul, 06120 Halle/Saale (DE); FIEDLER, Erik, 06120 Halle/Saale (DE)
(74) Representative: Haucke, Kerstin
(86) International application number: PCT/EP2021/054023
(87) International publication number: WO 2021/165395

(56) References cited:
- WO-A1-2018/029157
- FRIEDMAN M ET AL: "Phage display selection of Affibody molecules with specific binding to the extracellular domain of the epidermal growth factor receptor", PROTEIN ENGINEERING, DESIGN AND SELECTION, OXFORD JOURNAL, LONDON, GB, vol. 20, no. 4, 1 April 2007 (2007-04-01), pages 189-199, XP002530618, ISSN: 1741-0126, DOI: 10.1093/PROTEIN/GZM011 [retrieved on 2007-04-23]
- ALEKSANDRA SOKOLOWSKA-WEDZINA ET AL: "Efficient production and purification of extracellular domain of human FGFR-Fc fusion proteins from Chinese hamster ovary cells", PROTEIN EXPRESSION AND PURIFICATION, vol. 99, 1 July 2014 (2014-07-01), pages 50-57, XP055405142, SAN DIEGO, CA. ISSN: 1046-5928, DOI: 10.1016/j.pep.2014.03.012

## Description

### TECHNICAL FIELD

The present invention relates to the field of protein purification and relates in particular to novel proteins that bind to the soluble part of fibroblast growth factor receptor 3 (sFGFR3). The invention further relates to fusion proteins comprising novel proteins that bind to sFGFR3. In addition, the invention relates to affinity matrices comprising the sFGFR3 binding proteins of the invention. The invention also relates to a use of these sFGFR3 binding proteins or affinity matrices for affinity purification of sFGFR3 and to methods of affinity purification of sFGFR3 using the sFGFR3 binding proteins of the invention. Further uses relate to analytical methods for the determination of sFGFR3 in liquids.

### BACKGROUND OF THE INVENTION

Fibroblast growth factor receptor 3 (known as FGFR3), a receptor tyrosine kinase having an extracellular domain, a transmembrane domain, and an intracellular domain. FGFR3 is a cellular receptor capable of binding members of the fibroblast growth factor (FGF) family. The FGF binding site is located in extracellular immunoglobulin (Ig)-like domains II and III. Upon binding of FGF on the extracellular side, FGFR3 dimerization and autophosphorylation on tyrosine residues is promoted. The phosphorylation is essential for the stimulation of cell proliferation and required for interaction with other proteins. It is assumed that FGF binding to the extracellular side of FGFR3 results the induction of different signal cascades involved in proliferation, cell migration and differentiation. Mutations of FGFR3 were found in developmental disorders and cancer. For example, single dominant point mutations in FGFR3 result in constitutive activation of FGFR3 that impairs internalization and degradation of FGFR3, resulting in prolonged FGFR3 signaling with effects on, for example, bone development. This may be the cause for skeletal diseases, for example a severe disease with abnormal bone growth known as achondroplasia (short-limb dwarfism; ACH), thanatophoric dysplasia type I, thanatophoric dysplasia type II, hydrochondroplasia, severe achondroplasia with developmental delay and acanthosis nigricans, and FGFR3-related craniosynostosis such as Muenke syndrome and Crouzon syndrome with acanthosis nigricans. Constitutively activated FGFR3 may also be the cause for example for bladder cancer, cervical cancer, multiple myeloma, and others.

Naturally, a soluble form of the FGFR3 exists and circulates systemically. In this case, before a member of the FGF family can bind to the extracellular side of the cell-bound FGFR3, FGF may be captured by the soluble form of FGFR3 (referred to as sFGFR3 herein). Thereby, in the presence of sFGFR3, FGF cannot bind to FGFR3, the receptors do not dimerize and as a result, signal cascades are not induced. sFGFR3 is involved in the regulation of FGF and FGFR3 function and may thus contribute to certain FGFR3-related diseases. sFGFR3 therefore provides interesting medical options for several FGFR3-related diseases. New approaches using systemic administration of recombinant sFGFR3 for potential therapies for patients with FGFR3-related diseases such as achondroplasia were suggested. For medical uses, it is of great importance to provide highly purified sFGFR3. However, currently no methods for efficient purification of sFGFR3 exist.

Therefore, it is essential to provide methods for the purification of sFGFR3. There is an ongoing need in the art for advanced tools that allow an efficient sFGFR3 protein purification.

The present invention meets this need by providing novel binding proteins for sFGFR3. These novel sFGFR3 binding proteins are particularly advantageous because they allow a precise capturing in affinity chromatography.

The above overview does not necessarily describe all problems solved by the present invention.

### SUMMARY OF THE INVENTION

The present disclosure provides the following items 1 to 14, without being specifically limited thereto:
1. A binding protein for a soluble form of the fibroblast growth factor receptor 3 (sFGFR3) comprising an amino acid sequence with at least 89 % sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, or SEQ ID NO: 9. Some embodiments refer to a binding protein for a soluble form of the fibroblast growth factor receptor 3 (sFGFR3) comprising an amino acid sequence with at least 89 % sequence identity to SEQ ID NO: 1 or SEQ ID NO: 2.
2. The binding protein for sFGFR3 according to item 1, wherein the binding protein has a binding affinity of less than 300 nM for sFGFR3.
3. The binding protein for sFGFR3 according to any of items 1-2, wherein the binding protein has no detectable binding affinity for the Fc domain of immunoglobulin.
4. The binding protein for sFGFR3 according to item 1, wherein the protein comprises 2, 3, 4, 5, or 6 binding proteins linked to each other.
5. The binding protein for sFGFR3 according to item 4, wherein the binding protein is a homo-multimer, for example as shown in SEQ ID NOs: 11, 39-43, or a hetero-multimer, for example, as shown in SEQ ID NOs: 44-46.
6. A fusion protein comprising the binding protein according to any one of items 1-5, for example as shown in SEQ ID NO: 12-29, 39-46.
7. A fusion protein according to item 6, wherein the fusion protein comprises additionally a non-Immunoglobulin (Ig)-binding protein, preferably wherein the non-Ig-binding protein is defined by an amino acid sequence with at least 80 % identity to SEQ ID NO: 30 or SEQ ID NO: 36 or SEQ ID NO: 37.
8. A polynucleotide encoding the binding protein according to any one of items 1-5, or the fusion protein according to items 6-7.
9. The binding protein for sFGFR3 according to any one of items 1-5, or the fusion protein according to items 6-7, for use in affinity purification of sFGFR3.
10. An affinity separation matrix comprising a binding protein for sFGFR3 according to any one of items 1-5, or the fusion protein according to items 6-7.
11. Use of the binding protein for sFGFR3 according to any one of items 1-5, or the fusion protein according to items 6-7, or the affinity separation matrix according to item 10 for affinity purification of sFGFR3.
12. A method of affinity purification of a protein comprising sFGFR3, the method comprising: (a) providing a liquid that contains sFGFR3; (b) providing an affinity separation matrix comprising at least one binding protein for sFGFR3 according to any one of items 1-5 or a fusion protein according to items 6-7 coupled to said affinity separation matrix; (c) contacting said affinity separation matrix with the liquid under conditions that permit binding of the at least one binding protein for sFGFR3 according to any one of items 1-5 to sFGFR3 or of a fusion protein according to items 6-7; and (d) eluting said sFGFR3 from said affinity purification matrix.
13. Use of the binding protein for sFGFR3 according to items 1-5 or the fusion protein according to items 6-7, in methods to determine the presence of sFGFR3.
14. An *in vitro* method of analyzing the presence of sFGFR3 in liquid samples, the method comprising the following steps:
   (i) providing a liquid that contains sFGFR3,
   (ii) providing the binding protein for sFGFR3 according to items 1-5, or fusion protein or fusion protein according to items 6-7,
   (iii) contacting the liquid of (i) with the binding protein for sFGFR3 according to items 1-5 or fusion protein according to items 6-7 under conditions that permit binding of the at least one binding protein for sFGFR3 according to items 1-5 or fusion protein according to items 6-7 to sFGFR3,
   (iv) isolating the complex of sFGFR3 and binding protein for sFGFR3 according to items 1-5 or fusion protein according to items 6-7, and
   (v) determining the amount of the binding protein for sFGFR3 according to items 1-5 or the fusion protein according to items 6-7 in the liquid of (i).

This summary of the invention is not limiting, and other aspects and embodiments of the invention will become evident from the following description, examples and drawings. The scope of protection is defined by the claims.

### BRIEF DESCRIPTION OF THE FIGURES

**FIGURE 1****:** shows the amino acid sequences of sFGFR3-binding proteins of SEQ ID NO: 1, 2, 3, 4, 5, 6, 7, 8, and 9.
**FIGURE 2****:** shows binding to sFGFR3. **FIGURE 2A** shows an SPR analysis of the fusion protein comprising sFGFR3 binding protein of SEQ ID NO: 1 (CID204815) to immobilized sFGFR3. The KD is 75 nM. **FIGURE 2B** shows an SPR analysis of fusion protein comprising immobilized sFGFR3 binding protein of SEQ ID NO: 3 (CID206146) to sFGFR3. The KD is 2.64 nM.
**FIGURE 3****:** shows use of binding protein for sFGFR3 in affinity purification of sFGFR3.

The figure shows the DBC10% for sFGFR3 binding proteins. 20 mg/ml ligands were coupled to Praesto 85 epoxy activated resin and loaded with 1 mg/ml sFGFR3 at 6 min residence time. sFGFR3 was eluted with 100 mM citric acid, pH 3.5, followed by pH 2.0. CID206078 is a tetramer of SEQ ID NO: 1, all other proteins refer to fusion proteins:

| | |
|---|---|
| 206079 | (SEQ ID NO: 1)₂- (PAdeIFc)₂ |
| 206080 | PAdelFc - (SEQ ID NO: 1)₂- PAdelFc |
| 206082 | SEQ ID NO: 1 - PadelFc - SEQ ID NO: 2 - PAdelFc |
| 206083 | SEQ ID NO: 2 - PAdelFc - SEQ ID NO: 1 - PAdelFc |
| 204815 | PAdelFc - SEQ ID NO: 1 - PAdelFc |
| 206146 | PAdelFc - SEQ ID NO: 3 - PAdelFc |

PAdelFc as used in this experiment was a non-Fc binding protein as shown in SEQ ID NO: 30.

### DETAILED DESCRIPTION OF THE INVENTION

The present invention provides novel proteins having binding affinity for soluble part of FGFR3 (sFGFR3). The proteins of the present invention represent advanced and powerful tools that fill a gap in the field of protein engineering and purification. In particular, the novel proteins provide for an advantageous effect in protein purification by virtue of said binding affinity for soluble part of FGFR3. Thus, the novel proteins of the present invention are particularly advantageous because they allow precise capturing of sFGFR3 in affinity chromatography. The binding affinity for sFGFR3 is given by a polypeptide having SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, or SEQ ID NO: 9, or an amino acid sequence with at least 89 % identity to any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, or SEQ ID NO: 9. A common structural feature of the binding proteins is that they are based on artificial mosaic proteins that are stable under conditions as usually applied in affinity chromatography, for example, under alkaline conditions. For example, the general scaffold of the sFGFR3 binding protein is a triple helical scaffold. The artificial mosaic proteins are composed of fragments of several wild-type Protein A (SpA) domains and have further specific mutations that generate the unique and surprising sFGFR3 binding functionality.

Before the present invention is described in more detail below it is to be understood that this invention is not limited to the particular methodology, protocols and reagents described herein as these may vary. It is also to be understood that the terminology used herein is for the purpose of describing particular aspects and embodiments only and is not intended to limit the scope of the present invention, which is defined by the claims. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of developing new FGFR3-specific binding molecules for use in technical applications such as affinity chromatography, as well as in therapy and diagnostics.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the items, which follow, unless the context requires otherwise, the word "comprise", and variants such as "comprises" and "comprising", will be understood to imply the inclusion of a stated integer or step, or group of integers or steps, but not the exclusion of any other integer or step or group of integers or steps. The term "comprise(s)" or "comprising" may encompass a limitation to "consists of" or "consisting of", should such a limitation be necessary for any reason and to any extent.

All sequences referred to herein are disclosed in the attached sequence listing that, with its whole content and disclosure, forms part of the disclosure content of the present specification.

### General Definitions of Important terms used in the Application

The term "Fibroblast Growth Factor Receptor 3" ("FGFR3") as used herein, refers to any native or variant FGFR3 polypeptide. The wildtype FGFR3 receptor comprises an extracellular domain with three immunoglobulin-like domains (positions 23-375), a transmembrane domain (positions 376-396) and an intracellular (cytoplasmic) domain (397-806). The naturally occurring human FGFR3 protein has an amino acid sequence as shown in Genbank Accession number NP 000133, herein represented by SEQ ID NO: 31.

As used herein, the term "extracellular domain of FGFR3" (ECD) refers to the portion of a FGFR3 polypeptide that extends beyond the transmembrane domain into the extracellular space. The ECD of FGFR3 has an amino acid sequence ranging from positions 23 to 375 of FGFR3 (SEQ ID NO: 31) and is shown in SEQ ID NO: 32.

The terms "soluble domain of FGFR3" or "soluble part of FGFR3" or "soluble form of FGFR3" or "soluble FGFR3" or "sFGFR3" can be used interchangeably herein and refer to a polypeptide comprising the ECD or comprising or consisting of a variant of the ECD.

The terms "sFGFR3 binding protein" or "sFGFR3 binding polypeptide" or "binding protein for sFGFR3" may be used interchangeably herein to describe a protein that is capable to bind to the soluble part of FGFR3 (sFGFR3) (or variants thereof).

As described herein, a "sFGFR3 binding protein" refers to a protein with detectable interaction with sFGFR3 thereof, as for example determined by SPR analysis or other appropriate technology known to someone skilled in the art.

The terms "binding affinity" and "binding activity" may be used herein interchangeably and they refer to the ability of a polypeptide of the invention to bind to another protein, peptide, or fragment or domain thereof. Binding affinity is typically measured and reported by the equilibrium dissociation constant (K_{D}) which is used to evaluate and rank the strength of bimolecular interactions. The binding affinity and dissociation constants can be measured quantitatively. Methods for determining binding affinities are well known to the skilled person and can be selected, for instance, from the following methods that are well established in the art: surface plasmon resonance (SPR), enzyme-linked immunosorbent assay (ELISA), kinetic exclusion analysis (KinExA assay), Bio-layer interferometry (BLI), flow cytometry, fluorescence spectroscopy techniques, isothermal titration calorimetry (ITC), analytical ultracentrifugation, radioimmunoassay (RIA or IRMA), and enhanced chemiluminescence (ECL). Typically, the dissociation constant K_{D} is determined at temperatures in the range of 20°C and 30°C. If not specifically indicated otherwise, K_{D} values recited herein are determined at 25°C by SPR. The most widely used SPR-based system is the BIAcore, produced by BIAcore AB. In various embodiments of the present invention, the binding affinity for sFGFR3 may be determined by the BIAcore SPR system. In various other embodiments of the present invention, the polypeptide of the invention has a binding affinity for sFGFR3, as determined by SPR, wherein the concentration of the analyte in the SPR assay is, preferably between 0.1fold and 10fold of the affinity and wherein the binding affinity is determined at 25 °C.

The term "fusion protein" relates to a protein comprising at least a first protein joined genetically to at least a second protein. A fusion protein is created through joining of two or more genes that originally coded for separate proteins. Thus, a fusion protein may comprise a multimer of identical or different proteins which are expressed as a single, linear polypeptide.

As used herein, the term "linker" refers in its broadest meaning to a molecule that covalently joins at least two other molecules.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide, for example, to the polypeptide of SEQ ID NO: 1. Methods for sequence alignment are well known in the art. For example, for determining the extent of an amino acid sequence identity of an arbitrary polypeptide relative to the amino acid sequence of, for example, SEQ ID NO: 1, the SIM Local similarity program is preferably employed (Xiaoquin Huang and Webb Miller (1991), Advances in Applied Mathematics, vol. 12: 337-357), that is freely available. For multiple alignment analysis, ClustalW is preferably used (Thompson et al. (1994) Nucleic Acids Res., 22(22): 4673-4680).

The terms "protein" and "polypeptide" refer to any chain of two or more amino acids linked by peptide bonds and does not refer to a specific length of the product. Thus, "peptides", "protein", "amino acid chain", or any other term used to refer to a chain of two or more amino acids, are included within the definition of "polypeptide", and the term "polypeptide" may be used instead of, or interchangeably with, any of these terms. The term "polypeptide" is also intended to refer to the products of post-translational modifications of the polypeptide like, e.g., glycosylation, which are well known in the art.

The term "alkaline stable" or "alkaline stability" or "caustic stable" or "caustic stability" refers to the ability of the sFGFR3 binding protein of the invention to withstand alkaline conditions without significantly losing the ability to bind to FGFR3. The skilled person in this field can easily test alkaline stability by incubating a sFGFR3 binding protein with sodium hydroxide solutions, e.g., as described in the Examples, and subsequent testing of the binding activity to sFGFR3 by routine experiments known to someone skilled in the art, for example, by chromatographic approaches. The term "chromatography" refers to separation technologies which employ a mobile phase and a stationary phase to separate one type of molecules (e.g., sFGFR3) from other molecules (e.g., contaminants) in the sample. The liquid mobile phase contains a mixture of molecules and transports these across or through a stationary phase (such as a solid matrix). Due to the differential interaction of the different molecules in the mobile phase with the stationary phase, molecules in the mobile phase can be separated.

The term "affinity chromatography" refers to a specific mode of chromatography in which a ligand coupled to a stationary phase interacts with a molecule (i.e. sFGFR3) in the mobile phase (the sample) i.e. the ligand has a specific binding affinity for the molecule to be purified. As understood in the context of the invention, affinity chromatography involves the addition of a sample containing a sFGFR3 to a stationary phase which comprises a chromatography ligand, such as a sFGFR3 binding protein of the invention.

The terms "solid support" or "solid matrix" are used interchangeably for the stationary phase.

The terms "affinity matrix" or "affinity purification matrix" or "affinity chromatography matrix", as used interchangeably herein, refer to a matrix, e.g., a chromatographic matrix, onto which an affinity ligand e.g., a sFGFR3 binding protein of the invention is attached. The attached affinity ligand (e.g., sFGFR3 binding protein) is capable of specific binding to a molecule of interest (e.g., sFGFR3) which is to be purified or removed from a mixture.

The term "affinity purification" as used herein refers to a method of purifying sFGFR3 from a liquid by binding sFGFR3 to a sFGFR3 binding protein that is immobilized to a matrix. Thereby, other components of the mixture except sFGFR3 are removed. In a further step, the bound sFGFR3 can be eluted in highly purified form.

### Detailed description of the embodiments of the invention

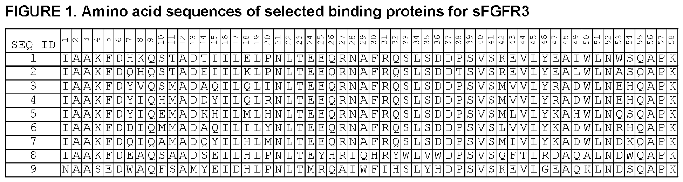

The novel proteins of the present invention including SEQ ID NOs: 1-9 exhibit a high binding affinity for sFGFR3 as determined by SPR. The sFGFR3 binding protein comprises, consists essentially of, or consists of an amino acid sequence of SEQ ID NOs: 1-9 or an amino acid with at least 89 % sequence identity thereto, respectively. In some embodiments, sFGFR binding proteins have amino acid sequences as shown in **FIGURE 1****.**

In some embodiments, the sFGFR3 binding protein has at least 89 % sequence identity to the amino sequence of SEQ ID NO: 1. In some embodiments, the sFGFR3 binding protein has at least 89 % sequence identity to the amino sequence of SEQ ID NO: 2. In some embodiments, the sFGFR3 binding protein has at least 89 % sequence identity to the amino sequence of SEQ ID NO: 3. In some embodiments, the sFGFR3 binding protein has at least 89 %, 91 %, 93%, 94%, 96 %, 98 %, or 100 % sequence identity to any of the amino sequences of SEQ ID NOs: 1-9. For example, "at least 89 % identity to amino acid of SEQ ID NO: 1" refers to 6 substitutions compared to amino acid SEQ ID NO: 1; "at least 95 % identity to amino acid of SEQ ID NO: 1" refers to 1 or 2 substitutions compared to amino acid SEQ ID NO: 1; and at least "98 % identity to amino acid of SEQ ID NO: 1" refers to 1 substitution compared to amino acid SEQ ID NO: 1.

One advantage of the disclosed sFGFR3 binding proteins is the functional characteristic that they bind specifically to sFGFR3. This is a particular advantage in the purification of sFGFR3. The sFGFR3 binding protein of the invention is functionally characterized by a binding affinity of less than 300 nM for sFGFR3. The sFGFR3 binding protein of the invention binds to sFGFR3 with a dissociation constant K_{D} below 300 nM, preferably below 200 nM, preferably below 100 nM, or more preferably below 50 nM, as shown in Example 3.

**Multimers.** In one embodiment of the invention, the sFGFR3 binding protein comprises 1, 2, 3, 4, 5, or 6 sFGFR3 binding proteins linked to each other. In some embodiments, the sFGFR3 binding protein can be, for example, a monomer, a dimer, a trimer, a tetramer, a pentamer, or a hexamer. In some embodiments, the sFGFR3 binding protein is a tetramer. Multimers of the protein of the invention are fusion proteins generated artificially, generally by recombinant DNA technology well-known to a skilled person. In some embodiments, the sFGFR3 binding protein is a homo-multimer, e.g. the amino acid sequences of sFGFR3 binding proteins are identical, e.g. as shown in SEQ ID NO: 11 (tetramer of SEQ ID NO: 1) and as follows:

IAAKFDHKQSTADTIILELPNLTEEQRNAFRQSLSDDPSVSKEVLYEAIWLNWSQAPK
IAAKFDHKQSTADTIILELPNLTEEQRNAFRQSLSDDPSVSKEVLYEAIWLNWSQAPK
IAAKFDHKQSTADTIILELPNLTEEQRNAFRQSLSDDPSVSKEVLYEAIWLNWSQAPK
IAAKFDHKQSTADTIILELPNLTEEQRNAFRQSLSDDPSVSKEVLYEAIWLNWSQAPK.

Other examples for homo-multimeric binding proteins are given in SEQ ID NOs: 39- 43.

In other embodiments, the sFGFR3 binding protein is a hetero-multimer, e.g. the amino acid sequences of sFGFR3 binding proteins are different, e.g. as shown in SEQ ID NOs: 44, 45, 46

**Fusion proteins.** According to one embodiment, provided herein is a fusion protein comprising one or more, for example two, sFGFR3 binding protein(s) as disclosed throughout the present specification. More specifically, the fusion protein comprises one or more sFGFR3 binding protein(s) as disclosed herein and a further polypeptide distinct from the polypeptide as disclosed. In various embodiments, the further polypeptide distinct from the sFGFR3 binding protein as disclosed herein might be a non-Ig-binding protein, for example but not limited to, a protein that does not bind to the Fc part of immunoglobulin. In some embodiments, a non-Ig binding protein has at least 80% identity to SEQ ID NO: 30, SEQ ID NO: 36, or SEQ ID NO: 37. In some embodiments, a non-Ig binding protein has at least 89 % identity to SEQ ID NO: 30, SEQ ID NO: 36, or SEQ ID NO: 37. In some embodiments, a non-Ig binding protein has at least 95 % identity to SEQ ID NO: 30, SEQ ID NO: 36, or SEQ ID NO: 37. Exemplary non-Ig binding proteins are the amino acid sequences of SEQ ID NO: 30, SEQ ID NO: 36, or SEQ ID NO: 37 )(also referred to as PAdelFc herein). Accordingly, some embodiments encompass fusion proteins comprising one or two sFGFR3 binding protein(s) as disclosed herein and one or two non-Ig-binding polypeptide(s. In some embodiments, for example in affinity purification applications, such constructs might be beneficial.

In some embodiments, a fusion protein may comprise the following combinations (from N-terminus to C-terminus):
(a) sFGFR3 binding protein - non-Ig binding protein;
(b) Non-Ig binding protein - sFGFR3 binding protein;
(c) Non-Ig binding protein - sFGFR3 binding protein - non-Ig binding protein (for example, see SEQ ID NOs: 12-29);
(d) sFGFR3 binding protein - non-Ig binding protein - non-Ig binding protein;
(e) sFGFR3 binding protein - sFGFR3 binding protein - non-Ig binding protein - non-Ig binding protein (for example, CID206079 (SEQ ID NO: 40) SEQ ID NO: 1 - SEQ ID NO: 1 - PAdelFc - PAdelFc; comparable construct in SEQ ID NO: 39; CID206081 (SEQ ID NO: 44): SEQ ID NO: 1 - SEQ ID NO: 2 - PAdelFc - PAdelFc);; SEQ ID NOs: 41-42: SEQ ID NO: 2 - SEQ ID NO: 2 - PAdelFc - PAdelFc;
(f) Non-Ig binding protein - non-Ig binding protein - sFGFR3 binding protein
(g) Non-Ig binding protein - sFGFR3 binding protein - sFGFR3 binding protein - non-Ig binding protein (for example CID206080 (SEQ ID NO: 43): PAdelFc - SEQ ID NO: 1 - SEQ ID NO: 1 - PAdelFc)
(h) sFGFR3 binding protein - Non-Ig binding protein - sFGFR3 binding protein - non-Ig binding protein (for example; CID206082 (SEQ ID NO: 45): SEQ ID NO: 1 - PAdelFc- SEQ ID NO: 2 - PAdelFc; for example, CID206083 (SEQ ID NO: 46): SEQ ID NO: 2 - PAdelFc - SEQ ID NO: 1 - PAdelFc).

Other combinations of non-Ig binding protein and sFGFR3 binding protein are also feasible to someone skilled in the art.

In some embodiments, a fusion protein comprises a sFGFR3 binding protein of SEQ ID NO: 1, or a sFGFR3 binding protein with at least 89 % amino acid identity thereto. In some embodiments, the fusion protein comprises a sFGFR3 binding protein of SEQ ID NO: 2, or a sFGFR3 binding protein with at least 89 % amino acid identity thereto. In some embodiments, the fusion protein comprises a sFGFR3 binding protein of any one of SEQ ID NOs: 3-9, or a sFGFR3 binding protein with at least 89 % amino acid identity thereto, respectively.

The moieties of the fusion protein may be linked to each other directly head-to-tail or may be linked by a linker, wherein the linker preferably is a peptide linker. In various embodiments, a peptide linker may be considered as an amino acid sequence which sterically separates the two portions of the fusion protein. Typically, such linker consists of between 1 and 10 amino acids. A fusion protein may be characterized as a protein formed by genetically fusing or combining a gene encoding the sFGFR3 binding protein with a gene encoding a polypeptide distinct from the polypeptide as described herein above. Accordingly, the fusion protein may be considered as the product of two or more genes that were translated together (no stop-codon in between).

**Molecules for purification or detection.** In some embodiments, the sFGFR3 binding proteins or fusion proteins comprising the sFGFR3 binding protein may also comprise additional amino acid residues at the N- and/or C-terminal end, such as for example an additional sequence at the N- and/or C-terminal end. Additional sequences may include for example sequences introduced e.g. for purification or detection. Typical examples for such sequences include, without being limiting, Strep-tags (see e.g. SEQ ID NO: 33), oligohistidine-tags, glutathione S-transferase, maltose-binding protein, inteins, intein fragments, or the albumin-binding domain of protein G, or others. In one embodiment, additional amino acid sequences include one or more peptide sequences that confer an affinity to certain chromatography column materials. The sFGFR3 binding protein or fusion protein comprising an sFGFR3 binding protein may include specific attachment sites for the attachment to solid supports, preferably at the C-terminal end, such as cysteine or lysine. Examples for sFGFR3 binding proteins with C-terminal Cysteine are shown in SEQ ID NOs: 11, 14, 15, 23-29, .

**Method of generation the sFGFR3 binding protein.** The present invention further provides a method for the generation of a novel sFGFR3 binding polypeptide as disclosed herein with binding affinity for sFGFR3, the method comprising the following steps: (i) providing a population of proteins; (ii) contacting the population of proteins of (i) with sFGFR3; (iii) identifying a complex comprising a sFGFR3 binding protein bound to sFGFR3; and (iv) obtaining a sFGFR3 binding protein which is capable of binding to sFGFR3.

The method for the generation of a novel sFGFR3 binding protein with binding affinity for a sFGFR3 may comprise, a further step of determining the binding affinity of the polypeptide to a sFGFR3. The binding affinity may be determined as described elsewhere herein.

**Use of the novel sFGFR3 binding proteins in technical applications.** Also provided herein is the use of any novel sFGFR3 binding protein of the present invention, including fusion proteins, in technical applications, preferably for use in affinity purification.

As described herein, affinity chromatography (also called affinity purification) makes use of specific binding interactions between molecules. Methods for immobilization of protein and methods for affinity chromatography are well-known in the field of protein purification and can be easily performed by a skilled person in this field using standard techniques and equipment.

In various embodiments, the method of affinity purification may further comprise one or more washing steps carried out under conditions sufficient to remove from the affinity purification matrix some or all molecules that are non-specifically bound thereto. Affinity purification matrices suitable for the disclosed uses and methods are known to a person skilled in the art.

Further embodiments relate to a process of manufacturing sFGFR3 comprising at least one chromatographic step employing an affinity chromatography matrix having an affinity for specifically binding sFGFR3 protein wherein the affinity ligand (binding protein) for sFGFR3 protein thereof as described above is coupled to said affinity chromatography matrix.

**Conjugation to a solid support.** In various aspects and/or embodiments of the present invention, the novel proteins disclosed herein including novel proteins generated or obtained by any of the methods as described above are conjugated to a solid support. In some embodiments of the invention, the polypeptide comprises an attachment site for site-specific covalent coupling of the polypeptide to a solid support. Specific attachment sites comprise without being limited thereto, natural amino acids, such as cysteine or lysine, which enable specific chemical reactions with a reactive group of the solid phase, or a linker between the solid phase and the protein.

**Affinity purification matrix.** In another embodiment, an affinity purification matrix is provided comprising a sFGFR3 binding protein, including a polypeptide identified by any of the methods as described above.

In preferred embodiments, the affinity purification matrix is a solid support. The affinity purification matrix comprises at least one sFGFR3 binding protein provided by the present invention. Accordingly, a novel sFGFR3 binding protein disclosed herein is encompassed for use in the purification of a protein by an affinity matrix.

Solid support matrices for affinity chromatography are known in the art and include, e.g., without being limited thereto, agarose and stabilized derivatives of agarose, cellulose or derivatives of cellulose, controlled pore glass, monolith, silica, zirconium oxide, titanium oxide, or synthetic polymers, and hydrogels of various compositions and combinations of the above.

The formats for solid support matrices can be of any suitable well-known kind. Such solid support matrix for coupling a novel protein or polypeptide of the present invention might comprise, e.g., one of the following, without being limited thereto: columns, capillaries, particles, membranes, filters, monoliths, fibers, pads, gels, slides, plates, cassettes, or any other format commonly used in chromatography and known to someone skilled in the art.

In one embodiment, the matrix is comprised of substantially spherical particles, also known as beads, for example Sepharose or Agarose beads. Matrices in particle form can be used as a packed bed or in a suspended form including expanded beds. In other embodiments of the invention, the solid support matrix is a membrane, for example a hydrogel membrane. In some embodiments, the affinity purification may involve a membrane as a matrix to which a sFGFR3 binding protein of the present invention is covalently bound. The solid support can also be in the form of a membrane in a cartridge.

In some embodiments, the affinity purification involves a chromatography column containing a solid support matrix to which a novel protein of the present invention is covalently bound. A novel protein or polypeptide of the present invention may be attached to a suitable solid support matrix via conventional coupling techniques. Methods for immobilization of protein ligands to solid supports are well-known in the field of protein engineering and purification and can easily be performed by a skilled person in this field using standard techniques and equipment.

Further, in some embodiments, the sFGFR3 binding protein as described herein or the fusion protein as described herein are used in methods to determine the presence of sFGFR3. Some embodiments relate to a method of analyzing the presence of sFGFR3 in liquid samples, the method comprising the following steps: (a) providing a liquid that contains sFGFR3, (b) providing the sFGFR3 binding protein, (c) contacting the liquid that contains sFGFR3 with the sFGFR3 binding protein as described herein under conditions that permit binding of the at least one sFGFR3 binding protein to sFGFR3, (d) isolating the complex of sFGFR3 and sFGFR3 binding protein, and (e) determining the amount of the sFGFR3 binding protein which indicates the amount of sFGFR3 in the liquid of (a). sFGFR3 binding protein includes a protein of at least 89 % identity to SEQ ID Nos: 1-9, or a fusion protein comprising a a protein of at least 89 % identity to SEQ ID Nos: 1-9

Further embodiments relate to a method of quantification of sFGFR3, the method comprising: (a) providing a liquid that contains sFGFR3; (b) providing a matrix to which sFGFR3 binding protein as described herein has been covalently coupled; (c) contacting said affinity purification matrix with the liquid under conditions that permit binding of the at least one sFGFR3 binding protein to sFGFR3; (d) eluting said sFGFR3; and optionally, (e) quantitating the amount of eluted sFGFR3. Methods to determine the presence of sFGFR3 in liquid samples might be quantitative or qualitative. Such methods are well known to the skilled person and can be selected, for instance but limited to, from the following methods that are well established in the art: enzyme-linked immunosorbent assay (ELISA), enzymatic reactions, surface plasmon resonance (SPR) or chromatography.

**Polynucleotides, vectors, host cells.** One embodiment covers an isolated polynucleotide or nucleic acid molecule encoding a sFGFR3 binding protein as disclosed herein (for example, SEQ ID NO: 35 or SEQ ID NO: 38). A further embodiment also encompasses proteins encoded by the polynucleotides as disclosed herein.

Further provided is a vector, in particular an expression vector, comprising the isolated polynucleotide or nucleic acid molecule of the invention, as well as a host cell comprising the isolated polynucleotide or the expression vector. For example, one or more polynucleotides, which encode a polypeptide as disclosed herein may be expressed in a suitable host and the produced protein can be isolated. A vector means any molecule or entity (e.g., nucleic acid, plasmid, bacteriophage or virus) that can be used for transfer of protein-encoding information into a host cell. Suitable vectors that may be applied in the present invention are known in the art. Furthermore, an isolated cell comprising a polynucleotide or nucleic acid or a vector as disclosed hereinis provided. Suitable host cells include prokaryotes or eukaryotes, for example a bacterial host cell, a yeast host cell or a non-human host cell carrying a vector. Suitable bacterial expression host cells or systems are known in the art. Various mammalian or insect cell culture systems as known in the art can also be employed to express recombinant proteins.

**Method of producing a protein of the invention.** In a further embodiment, a method for the production of the sFGFR3 binding protein as described is provided, the method comprising the step(s): (a) culturing a (suitable) host cell under conditions suitable for the expression of a sFGFR3 binding protein so as to obtain said sFGFR3 binding protein; and (b) optionally isolating said sFGFR3 binding protein. Suitable conditions for culturing a prokaryotic or eukaryotic host are well known to a person skilled in the art.

A sFGFR3 binding protein may be prepared by any conventional and well-known techniques such as plain organic synthetic strategies, solid phase-assisted synthesis techniques, or by commercially available automated synthesizers. They may also be prepared by conventional recombinant techniques, alone or in combination with conventional synthetic techniques.

In one embodiment, a method for the preparation of sFGFR3 binding is provided, as detailed above, said method comprising the steps: (a) providing a nucleic acid molecule encoding a sFGFR3 binding polypeptide; (b) introducing said nucleic acid molecule into an expression vector; (c) introducing said expression vector into a host cell; (d) culturing the host cell in a culture medium; (e) subjecting the host cell to culturing conditions suitable for expression of the sFGFR3 binding protein, thereby producing a sFGFR3 binding polypeptide; optionally (f) isolating the protein or polypeptide produced in step (e); and (g) optionally conjugating the protein or polypeptide to a solid matrix as described above. In various embodiments of the present invention the production of the sFGFR3 binding protein is performed by cell-free *in vitro* transcription and translation.

The following Examples are provided for further illustration of the invention. The invention, however, is not limited thereto, and the following Examples merely show the practicability of the invention on the basis of the above description.

### EXAMPLES

The following Examples are provided for further illustration of the invention. The invention, however, is not limited thereto, and the following Examples merely show the practicability of the invention on the basis of the above description.

### Example 1. Selection of sFGFR3 binding proteins

*Library construction and cloning of SEQ ID NOs: 1-2*. Libraries based on a non-Ig-binding protein as based on stable Protein A-like variants (artifical mosaic proteins composed of fragments of Protein A domains and additional mutations) comprising randomized amino acid positions were synthesized in house by randomized oligonucleotides generated by synthetic trinucleotide phosphoramidites (ELLA Biotech) to achieve a well-balanced amino acid distribution with simultaneously exclusion of cysteine and other amino acid residues at randomized positions. The corresponding cDNA library was amplified by PCR and ligated into a pCD33-OmpA phagemid. Aliquots of the ligation mixture were used for electroporation of *E. coli* SS320 (Lucigen) to produce and purify the phage library to store them as cryo-stocks. Unless otherwise indicated, established recombinant genetic methods were used.

*Selection of SEQ ID NOs: 1-2 by TAT phage display.* The naive library was enriched against biotinylated sFGFR3 using phage display as selection system (OFF-target: Sigmablocker). Competent bacterial *E. coli* ER2738 (Lucigene) were infected with the cryo phage library. Amplification and purification of the phage library was carried out using standard methods known to a skilled person. The selection in solution (SIS) method was utilized to allow binding between biotinylated sFGFR3 and the phage library in solution. The sFGFR3-phage complexes were captured by streptavidin/neutravidin magnetic SpeedBeads^{™} (Fisher Scientific). The sFGFR3 concentration during phage incubation was lowered from 200 nM (first round) to 100 nM (second round) to 40 nM (third round), and 10 nM (fourth round). A preselection with empty magnetic beads was performed in each round. After each selection round sFGFR3 bound phages were eluted by trypsin and reamplified using *E. coli* ER2738. To identify target specific phage pools, eluted and reamplified phages of each selection round were analysed by phage pool ELISA. Wells of a medium binding microtiter plate (Greiner Bio-One) were either precoated with streptavidin (10 µg/ml) followed by coating with biotinylated sFGFR3 (2.5 µg/ml) or directly coated with unmodified sFGFR3 (2.5 µg/ml), human IgG-Fc (2.5 µg/ml) or BSA (2.5 µg/ml). Bound phages were detected using α-M13 HRP conjugated antibody (GE Healthcare).

*Selection method:* selection in solution (SIS).

*ON-target:* sFGFR3 unmodified or biotinylated with either NHS-biotin in 30x excess or maleimid-biotin in 60x excess; concentration 200, 100, 40, 10 nM.

*OFF-target:* empty magnetic streptavidin/neutravidin SpeedBeads^{™} blocked with 10xSigmablocker.

*Cloning of target binding phage pools into an expression vector.* Selection pools showing specific binding to sFGFR3 in phage pool ELISA were amplified by PCR according to methods known in the art, cut with appropriate restriction nucleases and ligated into a derivative of the expression vector pET-28a (Merck, Germany) comprising an N- and C-terminal monomer, a cysteine and a StrepTag II sequence.

*Results:* Various phage display selection strategies resulted in specific signals for the unmodified and the biotinylated ON-target sFGFR3. Controls with BSA or human IgG-Fc showed no unspecific binding. Selected pools were subcloned and proceed to high throughput screening. 50 variants were selected for lab-scale production and purification.

*Library construction and cloning* of *SEQ ID NOs:* 3-9: Three cDNA libraries based on alkaline stable non-Ig-binding Protein A-like variants (artifical mosaic proteins) comprising randomized amino acid positions were synthesized by GeneArt (Thermo Fisher Scientific) by synthetic trinucleotide phosphoramidites to achieve a well-balanced amino acid distribution with simultaneously exclusion of cysteine and other amino acid residues at randomized positions. For the following selection process by ribosome display these cDNA libraries were supplemented with a T7 promoter region at the 5' end and a spacer region at the 3' end, respectively. Unless otherwise indicated, established recombinant genetic methods were used.

*Selection* of *SEQ ID NOs: 3-9 by ribosome display:* The cDNA libraries including the ribosome display regulatory elements were each transcribed into the corresponding RNA library followed by in vitro translation into a protein library. Those generated mRNA-ribosome-protein-ternary complexes were stable and thus suitable for selection. The ternary complexes were allowed to bind the biotinylated target (sFGFR3) while the target was not immobilized (SIS) on magnetic beads yet. Target concentration started at 200 nM (round 1) and declined each round to 2 nM (round 4). Selected pools of round 3 and 4 were amplified by PCR according to methods known in the art, cut with appropriate restriction nucleases and ligated into a derivative of the expression vector pET-28a (Merck, Germany) comprising an N- and C-terminal monomer, a cysteine and a StrepTag II sequence.

To identify target specific pools, pools of round 3 and 4 were analyzed by pool ELISA. Therefore subcloned pools were transformed in *E. coli* BL21(DE3), cultivated in 3 ml autoinduction media and the cells were harvested by three freeze/thaw cycles. Wells of a medium binding microtiter plate (Greiner Bio-One) were coated with sFGFR3 (2.5 µg/ml). Lysates were allowed to bind the target followed by several washing steps. Bound variants were detected via Streptactin-HRP. *Results:* all selection pools showed specific binding to the unmodified (non-biotinylated) sFGFR3 and were therefore proceeded to high throughput screening.

### Example 2. Expression and purification of sFGFR3 binding proteins

Hits as identified by selection as described in Example 1 were identified after screening and proteins produced in µ-scale (Phynexus).

sFGFR binding proteins (including fusion proteins) were expressed in *Escherichia coli* BL21(DE3) using a low copy plasmid system under regulation of a T7 promoter. Proteins were produced in soluble form after induction by lactose included in the medium (autoinduction medium). BL21 (DE3) competent cells were transformed with the expression plasmid, spread onto selective agar plates (kanamycin) and incubated overnight at 37°C. Precultures were inoculated from single colony in 3 ml 2xYT medium supplemented with 50 µg/ml kanamycin and cultured for 6 hours at 37 °C at 200 rpm in a conventional orbital shaker in culture tubes. Main cultures were inoculated with 3 mL of precultures in 300 ml ZYM-5052 (0.5 % glycerol, 0.2 % lactose, 0.05 % glucose, 0.5 % yeast extract, 1.0 % casamino acids, 25 mM Na₂HPO₄, 25 mM KH₂PO₄, 5 mM Na₂SO₄, 2 mM MgSO₄ and trace elements) that was supplemented with 50 µg/ml kanamycin in 1 L Erlenmeyer flasks. Cultures were transferred to an orbital shaker and incubated at 30 °C and 200 rpm. Recombinant protein expression was induced by metabolizing glucose and subsequently allowing lactose to enter the cells. Cells were grown overnight for approx. 17 hours to reach a final OD600 of about 2-4. Before the harvest, the OD600 was measured, samples adjusted to 0.6/OD600 were withdrawn, pelleted and frozen at -20 °C. To collect biomass cells were centrifuged at 12000 x g for 15 min at 22 °C. Pellets were weighed (wet weight). Cells were stored at -20 °C before processing.

Proteins with affinity tag were purified by affinity chromatography and size exclusion. After affinity chromatography purification a size exclusion chromatography (SE HPLC or SEC) has been performed using an Äkta system and a Superdex^{™} 200 HiLoad 16/600 column (GE Healthcare). The SEC column has a volume of 120 ml and was equilibrated with 2 CV. The samples were applied with a flow rate of 1 ml/min. Fraction collection starts as the signal intensity reaches 10 mAU. Homogenous species of all purified ligands were obtained; no aggregation was detected. Following SDS-PAGE analysis positive fractions were pooled and their protein concentrations were measured. Further analysis included SDS-PAGE, SE-HPLC and RP-HPLC. Protein concentrations were determined by absorbance measurement at 280 nm using the molar absorbent coefficient. Reversed phase chromatography (RP-HPLC) has been performed using a Dionex HPLC system and a PLRP-S (5 µm, 300 Å) column (Agilent). Fusion proteins of sFGFR3 binding protein and sFGFR3 binding protein were purified by the following strategy: Q-Sepharose FF 275 ml (pH 6) (buffer A: 20 mM BisTris, 1 mM EDTA pH 6; buffer B: 20 mM BisTris, 1 mM EDTA, 1 M NaCL pH 6), Phenyl Sepharose HP 236 ml (buffer A: 20 mM BisTris, 1 mM EDTA, 1 M (NH₄)₂SO₄ pH 6; buffer B: 20 mM BisTris, 1 mM EDTA, pH 6), Desalting Sephadex^{®} G-25 560 ml (buffer: 20 mM Citric acid, 150 mM NaCl, 1 mM EDTA, pH 6.0). All sFGFR3 binding proteins were successfully purified with yields of 4-31 mg protein per liter cell culture volume (see Table 1 and Table 2). Note: CID204815 is a fusion protein that comprises SEQ ID NO: 1. CID204783 is a fusion protein that comprises SEQ ID NO: 2. CID206078 is a tetramer of SEQ ID NO: 1. SEQ refers to the corresponding SEQ ID NO: for the sFGFR binding protein, PAdelFc refers to the non-Ig-binding protein of SEQ ID NO: 30, Cys = Cysteine.

**Table 1. Yield of FGFR3 binding proteins after purification (Strep-Tactin column and gelfiltration)**

| **CID** | **Fusion protein** | **Yield per liter** |
|---|---|---|
| 206113 | PAdelFc-SEQ9-PAdelFc-Cys | 5.4 mg |
| 206121 | PAdelFc-SEQ8-PAdelFc-Cys | 7.5 mg |
| 206136 | PAdelFc-SEQ7-PAdelFc-Cys | 6.8 mg |
| 206137 | PAdelFc-SEQ6-PAdelFc-Cys | 5.5 mg |
| 206141 | PAdelFc-SEQ5-PAdelFc-Cys | 8.5 mg |
| 206142 | PAdelFc-SEQ4-PAdelFc-Cys | 4.5 mg |
| 206146 | PAdelFc-SEQ3-PAdelFc-Cys | 7.8 mg |
| 204783 | PAdelFc-SEQ2-PAdelFc-Cys | 30.9 mg |
| 204815 | PAdelFc-SEQ1-PAdelFc-Cys | 6.2 mg |

**Table 2. Analysis of sFGFR3 binding proteins after purification**

| **CID** | **alias** | **Yield DSP total (mg)** | **Yield DSP mg/L culture** | **Yield DSP mg/g wet biomass** | **Solubility [%]** |
|---|---|---|---|---|---|
| 206078 | (SEQ1)₄-Cys | 1100 | 131.1 | 5.9 | 70 |
| 206079 | (SEQ1)₂-(PAdelFc)₂-Cys | 1150 | 127.95 | 5.1 | 100 |
| 206080 | PAdelFc-(SEQ1)₂-PAdelFc-Cys | 2560 | 284.4 | 12.5 | 90 |
| 206081 | SEQ1-SEQ2-(PAdelFc)₂-Cys | 1010 | 112.5 | 4.6 | 100 |
| 206082 | SEQ1-PAdelFc-SEQ2-PAdelFc-Cys | 830 | 92.6 | 3.7 | 100 |
| 206083 | SEQ2-PAdelFc-SEQ1-PAdelFc-Cys | 1000 | 222.9 | 9.9 | 90 |

### Example 3. Analysis of proteins by Surface Plasmon Resonance (SPR)

sFGFR3 was biotinylated with Sulfo-NHS-Biotin standard reagent and purified via size exclusion chromatography (Superdex 200). 500-1500 RU sFGFR3 (ON-ligand) was immobilized by injection on a Streptavidin coated CM-5 sensor chip (GE Healthcare). Alternatively, 500-1500 RU sFGFR binding protein (ON-ligand) was immobilized on a CM-5 sensor chip (GE Healthcare). The chip was equilibrated with SPR running buffer.

Upon binding, protein analyte was accumulated on the surface increasing the refractive index. This change in the refractive index was measured in real time and plotted as response or resonance units versus time. The analytes were applied to the chip in serial dilutions with a flow rate of 30 µl/min. The association was performed for 120 seconds and the dissociation for 360 seconds. After each run, the chip surface was regenerated with 30 µl regeneration buffer (10 mM Glycine) and equilibrated with running buffer.

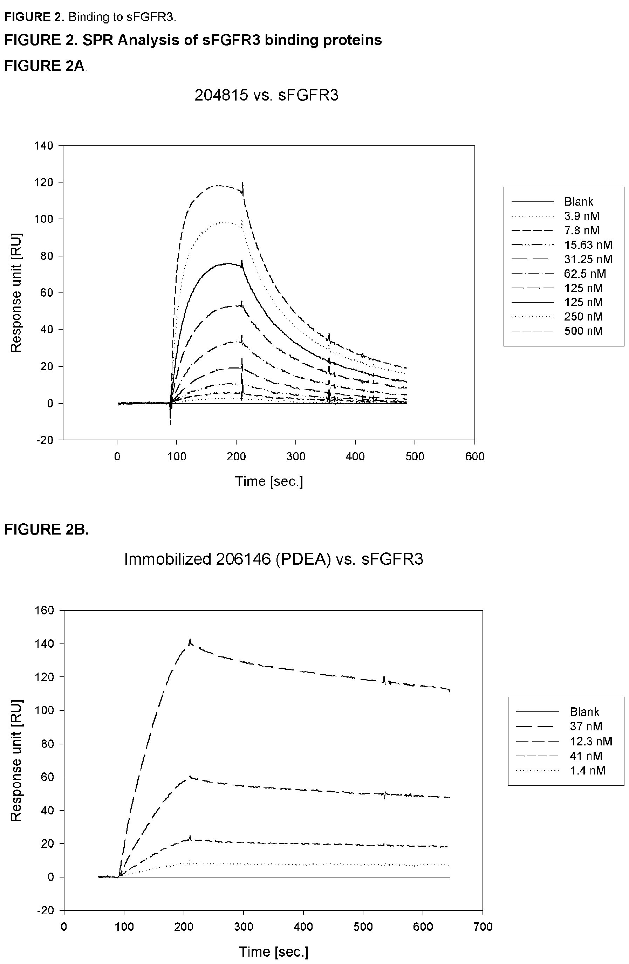

Binding studies were carried out by the use of the BIAcore 3000 (GE Healthcare); data evaluation was operated via the BIAevaluation 3.0 software, provided by the manufacturer, by the use of the Langmuir 1:1 model (RI=0). Evaluated dissociation constants (K_{D}) were standardized against the immobilized protein and indicated. Shown is the change in refractive index measured in real time and plotted as response or resonance unit [RU] versus time [sec]. Results are shown in **FIGURE 2** and in **Table 3 and in Table 4.** SEQ1 refers to sFGFR binding protein of SEQ ID NO: 1, SEQ2 refers to sFGFR binding protein of SEQ ID NO: 2, SEQ3 refers to sFGFR binding protein of SEQ ID NO: 3, etc. PAdelFc refers to the non-Ig-binding proteins of SEQ ID NO: 30, Cys = Cysteine.

**Table 3. Binding analysis of sFGFR binding proteins**

| **CID** | **SEQ ID** | **SPR vs. sFGFR3** | **SPR vs. IgG-Fc** |
|---|---|---|---|
| 206113 | 9 | 15 nM | No binding |
| 206121 | 8 | 15.7 nM | No binding |
| 206136 | 7 | 6.3 nM | No binding |
| 206137 | 6 | 6.4 nM | No binding |
| 206141 | 5 | 4.6 nM | No binding |
| 206142 | 4 | 4.5 nM | No binding |
| 206146 | 3 | 3.8 nM | No binding |

**Table 4. Affinities of fusion proteins (SPR analysis)**

| **CID** | **Fusion protein** | **Affinity [SPR analysis]** | | |
|---|---|---|---|---|
| | | **vs. sFGFR3** | **vs. hIgG-Fc** | |
| 206078 | SEQ1-SEQ1-SEQ1-SEQ1-Cys | 56.4 nM | no binding | |
| 206079 | SEQ1-SEQ1-PAdelFc-PAdelFc-Cys | 118 nM | no binding | |
| 206080 | PAdeIFc-SEQ1-SEQ1--PAdeIFc-Cys | 232 nM | no binding | |
| 206081 | SEQ1-SEQ2-PAdeIFc-PAdeIFc-Cys | 80.2 nM | no binding | |
| 206082 | SEQ1-PAdelFc-SEQ2-PAdelFc-Cys | 12.8 nM | no binding | |
| 206083 | SEQ2-PAdelFc-SEQ1-PAdelFc-Cys | 10 nM | no binding | |
| 204815 | PAdelFc-SEQ1-PAdelFc-Cys | 75 nM | no binding | |
| 204783 | PAdelFc-SEQ2-PAdelFc-Cys | 239 nM | no binding | |
| 206146 | PAdelFc-SEQ3-PAdelFc-Cys | | 4.2 nM | n.d. |

### Example 4. The sFGFR3 binding protein as affinity ligand for high efficient purification of sFGFR3

***Coupling efficiency.*** 20 mg purified sFGFR3 binding protein (CID206078; homotetramer of SEQ ID NO: 1, with c-terminal cysteine for site directed coupling) was immobilized per mL Praesto^{™} Epoxy 85 according to the manufacturer's instructions (coupling buffer: 50 mM Na₂HPO₄, 150 mM NaCl, 5 mM TCEP, 2.05 M Na₂SO₄ (or 1 M Na₂SO₄ per mL Resin), pH 8.5, coupling conditions: at 37°C for 16 h).

***DBC 10%.*** The resin with immobilized ligand was equilibrated in 1x PBS, pH 7.3. The dynamic binding capacity (DBC) was determined by the mass of 1 mg/ml injected sFGFR3 in 1x PBS pH 7.3 at 10 % breakthrough at 6 min residence time. The coupled resin showed good dynamic binding with capacity (DBC 10 %) of 10.7 mg/ml at 6 min residence time for the homo-tetramer (206078).

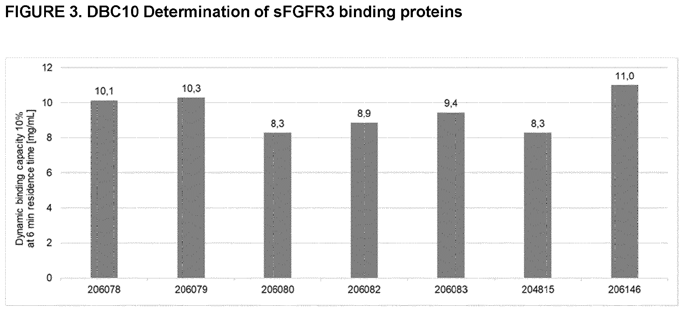

20 mg/ml sFGFR3 binding protein was coupled to the resin with immobilized sFGFR3 binding protein for 3 h at 35°C in the presence of 175 mg/ml crystalline Na₂SO₄, pH 8.5. 1 mg/ml sFGFR3 was loaded at 6 min residence time. The resin with immobilized sFGFR3 binding proteins showed good dynamic binding with capacity (DBC 10 %), see **FIGURE 3****.**

***Elution:*** sFGFR3 was eluted with 100 mM citric acid, pH 3.5, followed by 100 mM citrate acid, pH 2.0 (determination of elution ratio). The target sFGFR3 was completely eluted at pH 3.5. Similar results were obtained for resins with immobilized multimers comprising SEQ ID NO: 1 (e.g. SEQ ID NO: 11 CID206078 or SEQ ID NO: 40 CID206079).

***Stability (caustic stability*/*CIP*/*****reuses).*** CID206078 was coupled to Praesto^{™} Epoxy 85 as described above and treated with 0.1 M NaOH for at least 12.5 h (equivalent to 50 cycles at 15 min CIP each) at RT. The homo-tetramer of SEQ ID NO: 1 (CID206078) showed good caustic stability with about 90 % remaining capacity after NaOH treatment; the hetero-tetramer has caustic stability of about 85 % remaining capacity after NaOH treatment. Similar results were obtained for the resin Praesto^{™} Jetted Epoxy 50 (Purolite).

### Example 5. Leaching Assay using fusion proteins with comprising sFGFR3 binding protein

To determine low levels of leached binding protein in affinity chromatography is important for obtaining reliable results. Protein A ELISA Kits for the detection of native and recombinant Protein A (Repligen, Cat. No. 9000-1) were used for leaching assays according to manufacturer's instructions, except using 100 mM citric acid, pH 4.0 as assay buffer. Purified (tetramer of SEQ ID NO: 1 (CID206078) was tested. The tetramer can be detected using commercially available Protein A Leaching ELISA Kits. Leaching of the tetramer of SEQ ID NO: 1 is less than 40 ng per mg sFGFR3.

### Example 6. Purification of sFGFR3 from cell culture supernatant by affinity chromatography with sFGFR3 binding protein

Purified sFGFR3 binding protein (CID206078; homotetramer of SEQ ID NO: 1) was immobilized per mL Praesto^{™} Epoxy 85 according to the manufacturer's instructions. Cell culture supernatant was injected. The resin with immobilized CID206078 showed good dynamic binding with capacity (DBC 0.8 %) of 4.5 mg/ml at 6 min residence time. sFGFR3 was eluted with 100 mM citric acid, pH 3.5, followed by 100 mM citrate acid, pH 2.0. The target sFGFR3 was completely eluted at pH 3.5. Eluted fractions were neutralized. sFGFR3 could be completely extracted by affinity chromatography from cell culture supernatant.

## Claims

1. A binding protein for a soluble form of the fibroblast growth factor receptor 3 (sFGFR3) comprising an amino acid sequence with at least 89 % sequence identity to SEQ ID NO: 1 or SEQ ID NO: 2.

2. The binding protein for sFGFR3 according to claim 1, wherein the binding protein has a binding affinity of less than 300 nM for sFGFR3.

3. The binding protein for sFGFR3 according to any of claims 1-2, wherein the binding protein has no detectable binding affinity for the Fc domain of immunoglobulin.

4. The binding protein for sFGFR3 according to claim 1, wherein the protein comprises 2, 3, 4, 5, or 6 binding proteins linked to each other.

5. The binding protein for sFGFR3 according to claim 4, wherein the binding protein is a homo-multimer or a hetero-multimer.

6. A fusion protein comprising the binding protein according to any one of claims 1-5.

7. A fusion protein according to claim 6, wherein the fusion protein comprises additionally a non-Immunoglobulin (Ig)-binding protein, preferably wherein the non-Ig-binding protein is defined by an amino acid sequence with at least 80 % identity to SEQ ID NO: 30 or SEQ ID NO: 36.

8. A polynucleotide encoding the binding protein according to any one of claims 1-5, or the fusion protein according to claims 6-7.

9. The binding protein for sFGFR3 according to any one of claims 1-5, or the fusion protein according to claims 6-7, for use in affinity purification of sFGFR3.

10. An affinity separation matrix comprising a binding protein for sFGFR3 according to any one of claims 1-5, or the fusion protein according to claims 6-7.

11. Use of the binding protein for sFGFR3 according to any one of claims 1-5, or the fusion protein according to claims 6-7, or the affinity separation matrix according to claim 10 for affinity purification of sFGFR3.

12. A method of affinity purification of a protein comprising sFGFR3, the method comprising: (a) providing a liquid that contains sFGFR3; (b) providing an affinity separation matrix comprising at least one binding protein for sFGFR3 according to any one of claims 1-5 or a fusion protein according to claims 6-7 coupled to said affinity separation matrix; (c) contacting said affinity separation matrix with the liquid under conditions that permit binding of the at least one binding protein for sFGFR3 according to any one of claims 1-5 to sFGFR3 or of a fusion protein according to claims 6-7; and (d) eluting said sFGFR3 from said affinity purification matrix.

13. Use of the binding protein for sFGFR3 according to claims 1-5 or the fusion protein according to claims 6-7, in in vitro methods to determine the presence of sFGFR3.

14. An *in vitro* method of analyzing the presence of sFGFR3 in liquid samples, the method comprising the following steps:
(i) providing a liquid that contains sFGFR3,
(ii) providing the binding protein for sFGFR3 according to claims 1-5, or fusion protein or fusion protein according to claims 6-7,
(iii) contacting the liquid of (i) with the binding protein for sFGFR3 according to claims 1-5 or fusion protein according to claims 6-7 under conditions that permit binding of the at least one binding protein for sFGFR3 according to claims 1-5 or fusion protein according to claims 6-7 to sFGFR3,
(iv) isolating the complex of sFGFR3 and binding protein for sFGFR3 according to claims 1-5 or fusion protein according to claims 6-7, and
(v) determining the amount of the binding protein for sFGFR3 according to claims 1-5 or the fusion protein according to claims 6-7 in the liquid of (i).

## Patentansprüche

1. Bindungsprotein für eine lösliche Form des Fibroblastenwachstumsfaktor-Rezeptors 3 (sFGFR3), das eine Aminosäuresequenz mit mindestens 89 % Sequenzidentität zu SEQ ID NO: 1 oder SEQ ID NO: 2 umfasst.

2. Bindungsprotein für sFGFR3 nach Anspruch 1, wobei das Bindungsprotein eine Bindungsaffinität von weniger als 300 nM für sFGFR3 aufweist.

3. Bindungsprotein für sFGFR3 nach einem der Ansprüche 1-2, wobei das Bindungsprotein keine nachweisbare Bindungsaffinität für die Fc-Domäne von Immunglobulin aufweist.

4. Bindungsprotein für sFGFR3 nach Anspruch 1, wobei das Protein 2, 3, 4, 5 oder 6 miteinander verknüpfte Bindungsproteine umfasst.

5. Bindungsprotein für sFGFR3 nach Anspruch 4, wobei es sich bei dem Bindungsprotein um ein Homomultimer oder ein Heteromultimer handelt.

6. Fusionsprotein, das das Bindungsprotein nach einem der Ansprüche 1-5 umfasst.

7. Fusionsprotein nach Anspruch 6, wobei das Fusionsprotein zusätzlich ein Nicht-Immunglobulin(Ig)-Bindungsprotein umfasst, wobei das Nicht-Ig-Bindungsprotein vorzugsweise durch eine Aminosäuresequenz mit mindestens 80 % Identität zu SEQ ID NO: 30 oder SEQ ID NO: 36 definiert ist.

8. Polynukleotid, das das Bindungsprotein nach einem der Ansprüche 1-5 oder das Fusionsprotein nach den Ansprüchen 6-7 codiert.

9. Bindungsprotein für sFGFR3 nach einem der Ansprüche 1-5 oder Fusionsprotein nach den Ansprüchen 6-7 zur Verwendung bei der Affinitätsreinigung von sFGFR3.

10. Affinitätstrennungsmatrix, umfassend ein Bindungsprotein für sFGFR3 nach einem der Ansprüche 1-5 oder das Fusionsprotein nach den Ansprüchen 6-7.

11. Verwendung des Bindungsproteins für sFGFR3 nach einem der Ansprüche 1-5 oder des Fusionsproteins nach den Ansprüchen 6-7 oder der Affinitätstrennungsmatrix nach Anspruch 10 zur Affinitätsreinigung von sFGFR3.

12. Verfahren zur Affinitätsreinigung eines Proteins, das sFGFR3 umfasst, wobei das Verfahren Folgendes umfasst:
(a) Bereitstellen einer Flüssigkeit, die sFGFR3 enthält;
(b) Bereitstellen einer Affinitätstrennungsmatrix, die mindestens ein Bindungsprotein für sFGFR3 nach einem der Ansprüche 1-5 oder ein Fusionsprotein nach den Ansprüchen 6-7 gekoppelt an die Affinitätstrennungsmatrix umfasst;
(c) Inkontaktbringen der Affinitätstrennungsmatrix mit der Flüssigkeit unter Bedingungen, die die Bindung des mindestens einen Bindungsproteins für sFGFR3 nach einem der Ansprüche 1-5 an sFGFR3, oder eines Fusionsproteins nach den Ansprüchen 6-7, ermöglichen; und (d) Eluieren des sFGFR3 aus der Affinitätsreinigungsmatrix.

13. Verwendung des Bindungsproteins für sFGFR3 nach den Ansprüchen 1-5 oder des Fusionsproteins nach den Ansprüchen 6-7, in In-vitro-Verfahren zur Bestimmung des Vorhandenseins von sFGFR3.

14. *In-vitro*-Verfahren zum Analysieren des Vorhandenseins von sFGFR3 in Flüssigkeitsproben, wobei das Verfahren die folgenden Schritte umfasst:
(i) Bereitstellen einer Flüssigkeit, die sFGFR3 enthält,
(ii) Bereitstellen des Bindungsproteins für sFGFR3 nach den Ansprüchen 1-5 oder des Fusionsproteins oder des Fusionsproteins nach den Ansprüchen 6-7,
(iii) Inkontaktbringen der Flüssigkeit von (i) mit dem Bindungsprotein für sFGFR3 nach den Ansprüchen 1-5 oder dem Fusionsprotein nach den Ansprüchen 6-7 unter Bedingungen, die die Bindung des mindestens einen Bindungsproteins für sFGFR3 nach den Ansprüchen 1-5, oder des Fusionsproteins nach den Ansprüchen 6-7, an sFGFR3 ermöglichen,
(iv) Isolieren des Komplexes aus sFGFR3 und Bindungsprotein für sFGFR3 nach den Ansprüchen 1-5 oder Fusionsprotein nach den Ansprüchen 6-7, und
(v) Bestimmen der Menge des Bindungsproteins für sFGFR3 nach den Ansprüchen 1-5 oder des Fusionsproteins nach den Ansprüchen 6-7 in der Flüssigkeit von (i).

## Revendications

1. Protéine de liaison pour une forme soluble du récepteur de facteurs de croissance de fibroblastes 3 (sFGFR3) comprenant une séquence d'acides aminés comportant au moins 89 % d'identité de séquence avec la SEQ ID NO: 1 ou la SEQ ID NO: 2.

2. Protéine de liaison pour sFGFR3 selon la revendication 1, la protéine de liaison ayant une affinité de liaison de moins de 300 nM pour sFGFR3.

3. Protéine de liaison pour sFGFR3 selon l'une quelconque des revendications 1 et 2, la protéine de liaison n'ayant pas d'affinité de liaison détectable pour le domaine Fc d'immunoglobuline.

4. Protéine de liaison pour sFGFR3 selon la revendication 1, la protéine comprenant 2, 3, 4, 5 ou 6 protéines de liaison liées les unes aux autres.

5. Protéine de liaison pour sFGFR3 selon la revendication 4, la protéine de liaison étant un homo-multimère ou un hétéro-multimère.

6. Protéine de fusion comprenant la protéine de liaison selon l'une quelconque des revendications 1 à 5.

7. Protéine de fusion selon la revendication 6, la protéine de fusion comprenant de plus une protéine ne liant pas une immunoglobuline (Ig), préférablement la protéine ne liant pas une Ig étant définie par une séquence d'acides aminés comportant au moins 80 % d'identité de séquence avec la SEQ ID NO: 30 ou la SEQ ID NO: 36.

8. Polynucléotide codant pour la protéine de liaison selon l'une quelconque des revendications 1 à 5 ou la protéine de fusion selon les revendications 6 et 7.

9. Protéine de liaison pour sFGFR3 selon l'une quelconque des revendications 1 à 5, ou protéine de fusion selon les revendications 6 et 7, pour une utilisation dans une purification par affinité de sFGFR3.

10. Matrice de séparation par affinité comprenant une protéine de liaison pour sFGFR3 selon l'une quelconque des revendications 1 à 5, ou la protéine de fusion selon les revendications 6 et 7.

11. Utilisation de la protéine de liaison pour sFGFR3 selon l'une quelconque des revendications 1 à 5, ou de la protéine de fusion selon les revendications 6 et 7, ou de la matrice de séparation par affinité selon la revendication 10 pour une purification par affinité de sFGFR3.

12. Procédé de purification par affinité d'une protéine comprenant sFGFR3, le procédé comprenant : (a) la fourniture d'un liquide qui contient sFGFR3 ; (b) la fourniture d'une matrice de séparation par affinité comprenant au moins une protéine de liaison pour sFGFR3 selon l'une quelconque des revendications 1 à 5 ou une protéine de fusion selon les revendications 6 à 7 couplée à ladite matrice de séparation par affinité ; (c) la mise en contact de ladite matrice de séparation par affinité avec le liquide dans des conditions qui permettent une liaison de l'au moins une protéine de liaison pour sFGFR3 selon l'une quelconque des revendications 1 à 5 à sFGFR3 ou d'une protéine de fusion selon les revendications 6 et 7 ; et (d) l'élution dudit sFGFR3 de ladite matrice de purification par affinité.

13. Utilisation de la protéine de liaison pour sFGFR3 selon les revendications 1 à 5 ou de la protéine de fusion selon les revendications 6 et 7, dans des procédés *in vitro* pour déterminer la présence de sFGFR3.

14. Procédé *in vitro* d'analyse de la présence de sFGFR3 dans des échantillons liquides, le procédé comprenant les étapes suivantes :
(i) fourniture d'un liquide qui contient sFGFR3,
(ii) fourniture de la protéine de liaison pour sFGFR3 selon les revendications 1 à 5, ou d'une protéine de fusion ou d'une protéine de fusion selon les revendications 6 et 7,
(iii) mise en contact du liquide de (i) avec la protéine de liaison pour sFGFR3 selon les revendications 1 à 5 ou une protéine de fusion selon les revendications 6 et 7 dans des conditions qui permettent une liaison de l'au moins une protéine de liaison pour sFGFR3 selon les revendications 1 à 5 ou une protéine de fusion selon les revendications 6 et 7 à sFGFR3 ,
(iv) isolement du complexe de sFGFR3 et d'une protéine de liaison pour sFGFR3 selon les revendications 1 à 5 ou d'une protéine de fusion selon les revendications 6 et 7, et
(v) détermination de la quantité de la protéine de liaison pour sFGFR3 selon les revendications 1 à 5 ou de la protéine de fusion selon les revendications 6 et 7 dans le liquide de (i).
